# EUROPEAN PATENT APPLICATION

(11) **EP 1 057 892 A1**
(43) Date of publication of application: **06.12.2000**
(21) Application number: 99870111.4
(22) Date of filing: 02.06.1999
(51) Int. Cl.: C12N 15/31, C12N 1/21, C07K 14/245, A61K 39/108

(54) **Attenuated mutant enteropathogenic E. coli (EPEC) strains, process for their production and their use**

(71) Applicant: Centrum voor Onderzoek in Diergeneeskunde en Agrochemie, 1180 Brussel (Ukkel) (BE)
(72) Inventor: Marien, Jonas, 3000 Leuven (BE); Peeters, Johan, 9320 Erembodegem (BE)
(74) Representative: De Clercq, Ann

(57) **Abstract**

The present invention relates to attenuated mammalian enteropathogenic *E. coli* (EPEC) strains comprising a deletion of an intimin encoding gene sequence wherein said deletion fully eliminates a functional intimin protein, which can be effectively used for the production of a vaccine against enteropathogenic *E. coli* (EPEC) infections in mammals. The invention also relates to recombinant vectors to be used in methods for the construction of said to attenuated mammalian enteropathogenic *E. coli* (EPEC) strains. The invention also relates to a method for *in vitro* differentiating between field infected and vaccinated individuals wherein said vaccinated individuals have been vaccinated with a vaccine comprising attenuated mammalian enteropathogenic *E. coli* (EPEC) strains having a deletion of an intimin gene sequence and said field infected infected individuals are *in vitro* diagnosed on the basis of the presence of intimin antibodies.

SEQUENCE LISTING

<110> Centrum voor Onderzoek in Diergeneeskunde en Agroc

<120> Attenuated Mutant Enteropathogenic E. coli (EPEC)
      strains, process for their production and their use

<130> EP.99.CODA.EPEC

<140>
<141>

<160> 4

<170> PatentIn Ver. 2.1

<210> 1
<211> 31
<212> DNA
<213> Escherichia coli

<400> 1
aaaaaaggat ccaaaaatta cgccggaaga t                                31

<210> 2
<211> 31
<212> DNA
<213> Escherichia coli

<400> 2
aaaaaatcta gaaaaaggta aackaggtct c                                31

<210> 3
<211> 22
<212> DNA
<213> Escherichia coli

<400> 3
ccactcaaaa aactgtctgt tg                                          22

<210> 4
<211> 21
<212> DNA
<213> Escherichia coli

<400> 4
tccagtgaac taccgtcaaa g                                           21

## Description

The present invention concerns a process of obtaining attenuated mutant strains of enteropathogenic *Escherichia coli* (EPEC), derived from strains that carry partly or entirely the locus of enterocyte effacement which supplies them with certain pathogenic features. These features are, among others, effacement of the intestinal villi or brush border, rearrangement of the cytoskelet structure and resulting diarrhea. The invention equally concerns the attenuated strains that are the result of this process and their use in a vaccine.

EPEC strains are known as an important etiologic agent of human infant and animal diarrhea by adhering to the intestinal mucosa and producing the characteristic attaching and effacing (AE) lesion in the brush border microvillous membrane (26). They are also the prototype for a family of pathogens exhibiting this unique virulence mechanism (27). This group of attaching and effacing (AE) *Escherichia coli* includes enterohemorrhagic E.coli O157:H7, the mouse pathogen Citrobacter rodentium and the rabbit pathogen RDEC-1 (6, 22). EPEC strains are considered as the only important class of AE *E*. *coli* in rabbits (34). It was further demonstrated that rabbit EPEC type strain RDEC-1 possesses the same attributes as described for human EPEC strains (6, 17). Rabbit EPEC strains attach to intestinal tissue and cells and efface microvilli. Untill present no known thermostable or thermolable enterotoxins are described and the grand majority of these strains are not enteroinvasive (33a).

Next to the genetic features that can be more easily detected at present than before and the characteristical AE effect which are described more in detail further on, there are some additional characteristics which can be detected by using more traditional methods. Following the serological characterization of Salmonellae, the serotyping scheme for Escherichia coli strains was developed, originally based on three types of antigen: the somatic (O) antigen, the capsular (K) antigen and the flagellar (H) antigen. The somatic (O) antigens are lipopolysaccharide complexes and part of the cell wall structure of the E. coli. The polysaccharide repeating units give the O antigens their specific immunogenicity. A number of the O antigens cross-react serologically with or are even identical both chemically and serologically to somatic antigens of other organisms, like members of the Shigella and Salmonella groups. Some strains, called rough strains and designated OR, lack these repeating side chains and become auto-agglutinable. The capsular (K) antigens are mainly acidic polysaccharide. At current, K antigen typing is not regularly performed, except for some cases. The diversity of the flagellar (H) antigens is based on the different types of flagellin present as part of the flagellar structure. Many E. coli are either only slightly motile or non-motile on primary isolation. However, many strains on passage through a semisolid agar attain full motility. Those strains not developing motility are designated non-motile (NM) or H-. Thus this O:K:H type describes the serological features of E. coli in full. In general the K antigen is left off and a strain will be considered as serotyped by just detecting the O:H type. In some cases also the fimbrial (F) antigens are detected and in that case full typing of a strain results in a O:K:H:F designation.

There are indications that some serotypes are more likely to be associated with certain microenvironments. However, serotyping just labels the organisms without showing their specific pathogenicity or virulence factors. Certain serotypes appear to be linked with certain virulence traits (42). Long before their virulence characteristics were known, EPEC were first demonstrated on the basis of their serotype. In rabbit, there are a number of serotypes described as being more (O15, O26 and O103) or less (O128, O132) pathogenic. There is also one serotype, O109 which seems to be more pathogenic with suckling rabbits and less effective against weaned rabbits. Thus, serotyping gives us, in combination with a biotyping scheme, a first indication on the virulent properties of isolated rabbit EPEC strains.

A biotyping scheme based on the ability of strains to ferment dulcitol, raffinose, rhamnose and sorbose and to decarboxylate ornithine was shown to give optimal discrimination among rabbit EPEC strains. By applying this scheme, 20 biotypes could be established. With these biotypes, in combination with the serotype and motility of the strain, certain pathotypes can be distinguished. In general, strains that are designated O15:H-/3 (O15 positive, non-motile and belonging to biotype 3), O26:H+/4 and O103:H+/8 are to be considered as highly pathogenic for weaned rabbits. Other types like O132:H+/2 and O128:H+/2 include strains that have very different pathogenic features, from non-pathogenic to highly pathogenic but generaly only modestly pathogenic. Contrary to the afore mentioned strains, the O109:H+/1 type is more often associated with diarrhea in suckling rabbits and can be highly pathogenic to these infantile rabbits. Although more types can be isolated from diarrheal rabbits, the above mentioned pathotypes are the most important and the most frequently isolated in case of EPEC associated diarrhea with rabbits.

Apart from these more classic techniques, much work has been done on revealing the underlying genetic properties of EPEC, specifically human isolates. All genes necessary for AE are encoded on a 35kb chromosomal pathogenicity island called the locus of enterocyte effacement (LEE) (22). Among others, genes encoding a type III secretion system, secreted proteins (Esp) and the adhesin intimin were characterized and are situated in this island (1, 2, 11, 15, 16). Experiments confirmed that a cloned EPEC LEE is able to introduce the AE phenotype in an *E.coli* K12 host. Similar pathogenicity islands are found in all other AE pathogens, including those strains isolated from diarrheal rabbits (22, 41). More recently the translocated intimin receptor (Tir) has been described. This translocated receptor is secreted into the host cell's cytoplasm and serves as a receptor for intimin (18, 32). After an initial loose adherence, Esp proteins are secreted by the type III secretion system, some of which are transferred into the host cell cytoplasm (49). Transfer of Tir and positioning of this receptor in the host cell membrane allows EPEC to adhere intimatily by binding of intimin to its recpetor. Subsequently, association of the bundle forming pili (Bfp) from multiple EPEC cells are believed to make a rapid colonisation of the surrounding region possible. Underneath the attached EPEC, cytoskeletal rearrangements that are induced by the attaching cell result in localized degeneration of the epithelial brush border and the AE lesion, also described as pedestal formation (9, 27, 28). Resulting tissue damage leads to direct nutrient losses and a diminished food uptake. Bfp are plasmid encoded together with a positive virulence factor regulator (Per) and are not situated in the LEE locus. However , this plasmid is not necessary for the resulting pedestal formation (13, 14, 19).

The outer membrane protein intimine is transcribed from the *eae* gene and is considered as an important protein for the virulence mechanism. Mutants deffective in *eae* form immature AE lesions and do not induce reorganization of the cytoskeletal structure underneath the locus of infection (8). Immune response against the intimin protein is considered as a major immunoprotective factor against EPEC infection (8, 47). Deletion mutagenesis of *eae* in a human EPEC strain resulted in a strain that caused diarrhea in 4 of 11 of the volunteers that received this strain. Furthermore, an experimental infection later in time with a fully virulant EPEC strain of individuals that were experimentally vaccinated with the mutant strain demonstrated that application of such a vaccine failed to induce a long-term protection (8,10).

The present inventors have extensively studied EPEC as a causative agent of diarrhea in weaned and suckling rabbits. EPEC strains were identified as an important factor in rabbit enteropathy (39). Pathogenic properties and the abscence of thermostable or thermolabile enterotoxins were described (33a). Experimental infections with strains isolated from newborn, suckling rabbits and from weaned rabbits were perfomed and clinical studies revealed findings that were similar to the ones described in human EPEC infections (33, 35). Strains first attach to the Peyer's patche dome epithelium and later to the enterocytes of distal small intestine, cecum and colon. Effacement of the microvilli was reported (36). AF/R1 is an adhesine which has been reported to have some importance for RDEC-1, a laboratory isolated O15:H-/3 strain, to attach to Peyer's patches (7). However, AF/R1 or homologues did not seem to contribute to the pathogenicity of 40 strains of various serotypes that were negative in DNA hybridization with an AF/R1 specific probe, while these strains were all isolated from diarrheal rabbits and possesed clear virulence (41).

At present, there is only one solution for a rabbit breeder when colibacillosis arises: treating the animals with antibiotics and eventually eliminating the animals and repopulating the farm. However, antibiotic resistance is increasing and no guarantee is given that new animals are completely free of EPEC strains. No vaccines that are both safe and effective are currently available. Some experiments have been performed on using formaline-killed strains as possible vaccines against infection with O103 strains (5, 23), without directly useful results. Also naturally isolated strains that carried only some antigens of the pathogenic O103 serogroup have been tested as a vaccine, still without a safe protection against infection with a wild-type strain (25). More recently, the use of a strain mutated in the *sep* region (responsible for the type III secretion system) has been described (4a). Good results were obtained, but the strain was attenuated by using transposon mutagenesis and carries a kanamycine resistance gene, which makes it unsuitable for practical use. The strain, its method of obtaining and the eventual use of it has been described in a French patent application published under number 2 758 568 (24). In a more recent publication, the same researchers report the construction of similar strains, mutated in *espA, espB, espD* and *eae* respectively (29). As been reported before, strains mutated this way did not induce attaching and effacing (1, 2, 8, 10, 20, 45). In the *eae* mutant O103 strain, a slow cytopathic effect (CPE) was still noticed. This effect was abolished in the *esp* mutants and in earlier reported AF/R2 mutants. AF/R2 is an adhesin that has been reported before and for which there is a homologue present in O15:H- strains (3, 12). All of these are still constructed by making use of an antibiotic resistance marker gene which makes them not suitable for use in practice. Thus, the results of testing a human *eae* deletion mutant (8,10) and a rabbit secretion *(sep)* mutant (4a) as possible vaccine strains indicated that keeping the *eae* gene intact is necessary, as intimin is considered as an important antigen for inducing a protective immune response.

It is thus an aim of the present invention to provide attenuated mutant rabbit enteropathogenic *E*. *coli* strains which can be effectively used as a vaccine in mammals, preferably in non-human mammals.

It is another aim of the present invention to provide a method for producing an attenuated mutant rabbit enteropathogenic *E. coli* strain which can be effectively used as a vaccine in mammals, preferably in non-human mammals.

It is another aim of the present invention to provide a recombinant vector useful for producing an attenuated mutant rabbit enteropathogenic *E*. *coli* strain which can be effectively used as a vaccine in mammals, preferably in non-human mammals.

It is another aim of the present invention to provide a vaccine composition against enteropathogenic *E*. *coli* infections in mammals, preferably in non-human mammals.

It is another aim of the present invention to provide a method for *in vitro* differentiating between enteropathogenic *E*. *coli* field infected and vaccinated mammals, preferably in non-human mammals.

The present invention relates more particularly to an attenuated mammalian enteropathogenic *E*. *coli* (EPEC) strain comprising a deletion of an intimin encoding gene sequence wherein said deletion fully eliminates a functional intimin protein. Although deletion mutagenesis of *eae* gene sequences in human EPEC strains resulted in a strain that gave only slight protection against experimental infection, the present inventors surprisingly found that deletion of an *eae* gene sequence downstream of the suspected transmembrane anchor region of intimin in mammalian EPEC strains resulted in an effective protection against experimental infection. Preferably said mammalian strain is a non-human mammalian strain.

Preferably, the present invention relates to an attenuated rabbit enteropathogenic *E*. *coli* (EPEC) strain comprising a deletion of an intimin encoding gene sequence wherein said deletion fully eliminates a functional intimin protein.

According to a preferred embodiment, the present invention provides an attenuated rabbit enteropathogenic *E. coli* (EPEC) strain wherein said deletion consists of a deletion of an internal 248 basepair region of the *eae* gene. Said deletion should be such that it fully eliminates a functional intimin protein. The deleted region is situated downstream of the transmembrane anchor of intimin and it cuts out of frame, the eventually translated sequences downstream the deleted region will not be functional anymore.

The present invention also provides an attenuated rabbit enteropathogenic *E. coli* (EPEC) as deposited under No LMG P-18935 on April 22, 1999 in the Belgian Coordinated Collections of Microorganisms LMG collection.

According to another embodiment, the present invention relates to a recombinant vector comprising a deletion of an intimin encoding gene sequence as defined above, wherein said deletion fully eliminates the expression of functional intimin protein.

More particularly, the present invention relates to a vector as defined above wherein said deletion consists of a deletion of an internal region of the *eae* gene.

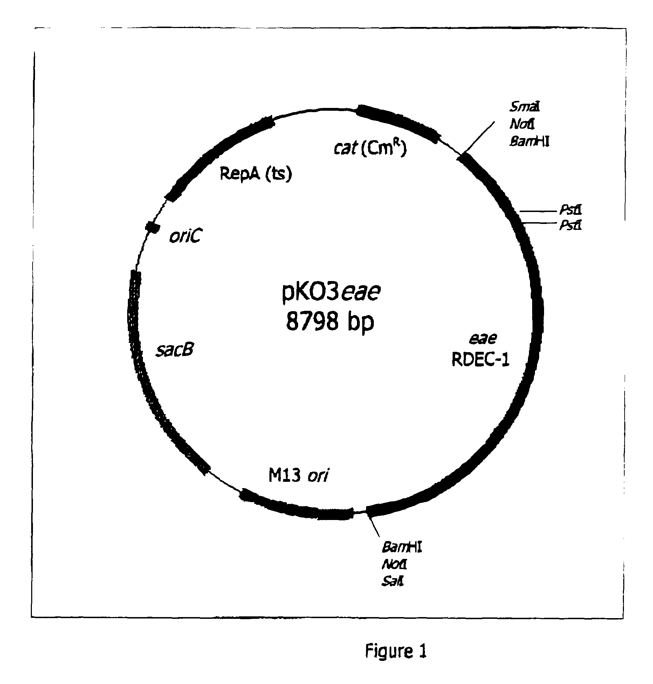

Even more particularly, the present invention relates to a vector as defined above termed pKO3Δ*eae* and characterized by a restriction map as shown in Figure 1.

According to another embodiment, the present invention relates to a method for producing an attenuated mammalian enteropathogenic *E*. *coli* (EPEC) strain as defined above comprising the step of introducing an *eae* gene deletion in a wild-type EPEC strain by homologous recombination using a vector comprising a deletion of an intimin encoding gene sequence as defined above. Preferably said mammalian strain is a non-human mammalian strain.

According to another embodiment, the present invention relates to a method for producing an attenuated rabbit enteropathogenic *E*. *coli* (EPEC) strain as defined above comprising the step of introducing an *eae* gene deletion in a wild-type EPEC strain by homologous recombination using a vector comprising a deletion of an intimin encoding gene sequence as defined above.

According to yet another embodiment, the present invention relates to an immunogenic composition comprising attenuated mammalian enteropathogenic *E*. *coli* (EPEC) strain comprising a deletion of an intimin encoding gene sequence as defined above. Preferably said mammalian strain is a non-human mammalian strain.

According to yet another embodiment, the present invention relates to an immunogenic composition comprising attenuated rabbit enteropathogenic *E*. *coli* (EPEC) strain comprising a deletion of an intimin encoding gene sequence as defined above.

According to another embodiment, the present invention relates to a live vaccine composition which provides protective immunity against an EPEC infection in a mammal (preferably a non-human mammal) comprising attenuated mammalian enteropathogenic *E*. *coli* (EPEC) strain comprising a deletion of an intimin encoding gene sequence as defined above. Preferably said mammalian strain is a non-human mammalian strain.

According to another embodiment, the present invention relates to a live vaccine composition which provides protective immunity against an EPEC infection in a rabbit comprising attenuated rabbit enteropathogenic *E*. *coli* (EPEC) strain comprising a deletion of an intimin encoding gene sequence as defined above.

If necessary, however, attenuated mammalian enteropathogenic *E. coli* (EPEC) strain may also be administered by any other route known in the art to aid the treatment of enteropathogenic *E*. *coli* (EPEC) infections.

According to a preferred embodiment, said vaccine composition is an oral vaccine.

Administration of the attenuated attenuated enteropathogenic *E. coli* (EPEC) strains of the invention may be performed by adding any adjuvant or any other immune response exhancing agent known in the art.

According to another embodiment, the present invention relates to a method for *in vitro* differentiating between field infected and vaccinated individuals wherein said vaccinated individuals have been vaccinated with a vaccine composition as defined above and said field infected infected individuals are *in vitro* diagnosed on the basis of the presence of intimin antibodies.

According to a preferred embodiment, the present invention relates to a method for *in vitro* differentiating between field infected and vaccinated individuals wherein the differentiation between wild type and mutant strain is made by using SEQ ID Numbers 3 and 4 in an amplificiation reaction.

According to another embodiment, the present invention relates to the use of an attenuated mammalian enteropathogenic *E*. *coli* (EPEC) strain comprising a deletion of an intimin encoding gene sequence as defined above for the preparation of a vaccine. Preferably said mammalian strain is a non-human mammalian strain.

According to another embodiment, the present invention relates to the use of an attenuated rabbit enteropathogenic *E*. *coli* (EPEC) strain comprising a deletion of an intimin encoding gene sequence as defined above for the preparation of a vaccine.

The contents of all references cited herein are incorporated by reference into the present text.

### FIGURE LEGENDS

Figure 1 : pKO3*eae* (8798 bp) results from the cloning of the *eae* sequence from RDEC-1 into the *Bam*HI restriction site of pKO3. Apart from the restriction sites for *Sma*l*, Not*l*, Bam*HI*, Eco*RI*, Pst*l and *Sal*l, the following features are shown : *cat* (chloramfenicol acetyltransferase), M13 *ori* (f1 (+) filamentous origin), *sac*B *(Bacillus subtilis sacB* gene), *ori*C*,* RepA (pSC101 tempereture sensitive). Cutting this vector with Pstl and re-ligating results in plasmid pKO3Δ*eae* having a deletion of the *eae* gene.
Figure 2 : PCR products from REPEC 015 :H-Δ*eae* (lane 1), wild type 97/241.6 (lane 2), a negative control (water, lane 3) and a DNA size marker (λ DNA gel cut with *Pst*l) are separated on a gel (0,8% agarose in a tris-borate ethylene diamine tetra-acetate (TBE) buffer). The primers used (SEQ ID NO 3 and SEQ ID NO 4) align upstream and downstream the 248 bp deleted region in *Δeae,* resulting in a 350 bp product for an uncut *eae* (lane 2) and in a 102 bp product for the *Δeae* recombinant gene (lane 1).
Figure 3 : The result of a Western blot from isolated membrane proteins detected by applying available polyclonal serum against rabbit EPEC (47) is shown. The technique used is described elsewhere (40, 47).
   Lane 1 shows the Biotinylated Broad Standard (Bio-Rad), lane 2 proteins from REPEC O15:H-Δ*eae* and lane 3 proteins from wild type strain 97/241.6. REPEC O15:H-Δ*eae* seems to have lost the intimin band (94kDa, arrow), while 97/241.6 still produces this band.
Figure 4 : Pane A shows the intimate and the effacing of microvilli by wild type strain 97/233.10 (3500X scanning electron microscope, SEM). Pane B shows identical effects observed after infection with wild type strain 97/241.6 (3500X). Pane C shows an overview of the same sample (900X) with a clear difference between uninfected and infected regions.
Figure 5: A scanning electron microscope picture is shown taken after the infection of a rabbit with the attenuated REPEC 015 :H-Δ*eae* strain (900X, SEM). No attachment of effacement by this strain is observed.
Figure 6 : Graphical presentation showing the daily feed uptake as measured and calculated three times a week. A significant difference is found between the non vaccinated and vaccinated/control group (confidence interval 95%).

### EXAMPLES

The invention described will be better understood by the following non limitating descriptions of experiments.

### Target strain selection

Biotyping was performed on a collection of putative rabbit EPEC strains available in the inventors' laboratory, by using routine methods (30, 38). Motility, indol reaction en ornithine decarboxylase activitity are tested on MIO tubes (GIBCO, Paisley, Schotland) following the manufacturer's specifications. Serotyping was performed by slide agglutination with specific antisera (31). Typing was repeated after safely storing the strains in a -70°C freezer. The presence of the locus of enterocyte effacement was detected by routine colony hybridization with non-radioactive probes A,B,C and D (22, 44). These probes cover the whole LEE genetic locus. Hybridization was performed at 65°C and Standard buffer was chosen as hybridisation buffer (46). Based on these findings, two O15:H- strains were chosen as reference strains for the pathogenic O15:H- rabbit EPEC group, namely 97/223.10 and 97/241.6. These strains are available upon request from Centrum voor Onderzoek in Diergeneeskunde en Agrochemie, Groeselenberg 99, B-1180 Brussel (Ukkel), Belgium.

### Construction and analysis of a mutant EPEC strain

The *eae* genes of 97/241.6, 97/223.10 and RDEC-1 were cloned by using standard techniques described in (44). Based on the published sequence of RDEC-1 *eae* (4), primers were chosen and PCR was performed on chromosomal DNA of the respective strains (primer up: 5' AAAAAAGGATCCAAAAATTACGCCGGAAGAT 3' (SEQ ID NO 1); primer down: 5' AAAAAATCTAGAAAAAGGTAAACKAGGTCTC 3' (SEQ ID NO 2); 10 cycles 15" 94°C, 30" 55,4°C, 2' 68°C followed by 20 cycles 15" 94°C, 30" 55,4°C, 2' 68°C + 20" each cycle). Expand™ polymerase (Boehringer Mannheim GmbH, Mannheim, Germany) was used and reactions were run on a Perkin Elmer GeneAmp 9600 Thermal Cycler. PCR products were cloned by using the pCR2.1-TOPO vector cloning system (Invitrogen, Groningen, The Netherlands). The RDEC-1 *eae* gene was cut out of the pCR2.1-TOPO plasmid with *Bam*HI and ligated in equallly cut pKO3 (21), resulting in plasmid pKO3*eae* (Figure 1). Correct ligation was checked by restriction digest and gel electrophoresis and by PCR (following the procedure described above, with recombinant plasmid as template DNA). An internal 248 basepair (bp) region of *eae* was removed by digesting pKO3*eae* with *Pst*l and self-ligating the plasmid, resulting in pKO3Δ*eae*. A PCR reaction test with primers flanking the deleted 248 bp region was designed (primer up 5' CCACTCAAAAAACTGTCTGTTG 3' (SEQ ID NO 3); primer down 5' TCCAGTGAACTACCGTCAAAG 3' (SEQ ID NO 4); 25 cycles 15" 94°C, 58,1°C, 72°C), making the detection of correctly ligated pKO3Δ*eae* easier. In case the 248 bp area was not cut out, the resulting product should be 350 bp and in case of a correct deletion 102 bp.

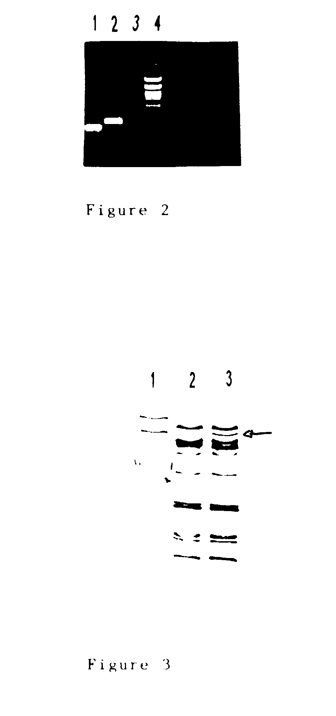

Plasmid pKO3 is a suicide plasmid with a temperature sensitive origin of replication (*ori*) and a saccharose sensitivity gene *(sacB)* from *Bacillus subtilis* as its mean feaures (21). This combination allowed it to be used as a tool for site-specific mutagenesis by promoting two succeeding homologuous recombinations. The vector was originally designed as a helper tool to analyze unidentified open reading frames of *Escherichia coli,* also known as genomics research (21). Strains 97/241.6 and 97/223.10 were made electrocompetent following routine techniques (44) and were electroporated (conditions used: 200Ω, 25 F, 1.80kV, using a 0,1 cm cuvette in a Bio-Rad Gene Pulser). Electroporated strains were grown in SOC broth for 30 minutes in a shaking incubator and were streaked out on agar plates as described by (21). Double homologuous recombination did not succeed, but following the method described by (21) in which the vector pKO3Δ*eae* first cointegrates with *eae* in the chromosome and then undergoes a second recombination, did result in numerous colonies. The second recombination event resulted in either the intact *eae* gene or *Δeae.* Since the deleted region was situated close to the N terminal end of *eae* (405 bp from the start codon) and further from the C terminal region (2166 bp from the stop codon), a recombination leaving *Δeae* was less favourable. Possible mutant colonies were picked up and tested by the PCR detection test (see Figure 2) described above or by colony hybridization using the same conditions as described above but with the 248 bp region as a probe (made by performing PCR with the flanking primers on the wild type strains' chromosomes and by cutting the resulting product with *Pst*l*,* resulting in the deleted 248 bp region probe). In this way, 465 strains originating from 97/223.10 have been tested and were all negative. 294 strains originating from 97/241.6 have been tested and only one colony that lost the 248 bp region has been isolated, resulting in the mutant strain REPEC O15:H- *Δeae* deposited under No. LMG P-18935 on April 22, 1999 in the Belgian Coordinated Collections of Microorganisms LMG collection.

### Protein analysis of the mutant strain

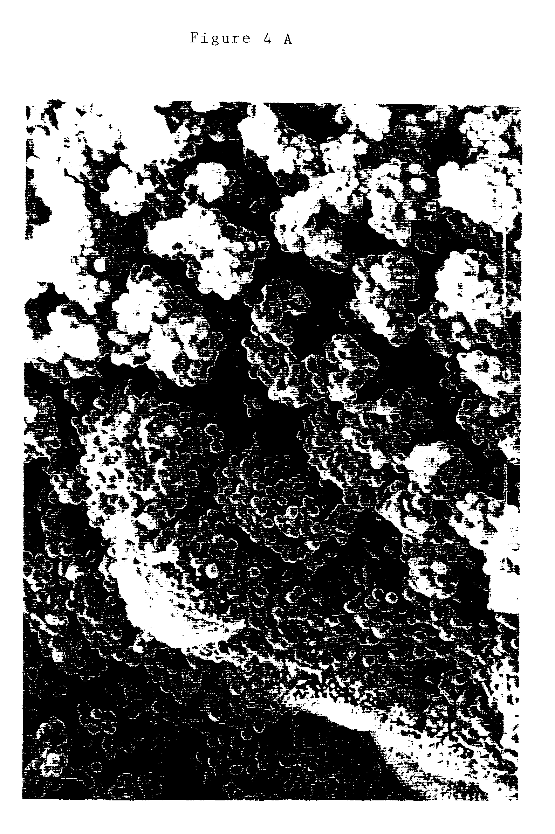

Hyperimmune serum against a O109:H+ strain, obtained in earlier described experiments (47), was used to visualize antigenic proteins of the REPEC O15:H- *Δeae* and 97/241.6 strains by immunoblotting performed as described before (40). The band of 94 kilodalton (kDa) size which has been identified as intimin was no longer visible in the lane where proteins of REPEC O15:H- *Δeae* were loaded (see Figure 3). This allowed the inventors to conclude that intimin was absent in the mutant strain, as expected.

### Pathogenic properties of mutant and non-mutant strains

Pathogenic properties of 97/241.6, 97/223.10 and REPEC O15:H- *Δeae* strain were tested by performing an *in vivo* infection experiment. Two New Zealand White MDL (Minimum Disease Level) rabbits were tested per strain, two control animals were housed separately and were never infected. These rabbits were purchased from N.V. Verlabreed (Nevele, Belgium) a breeder with well known hygienic status. Their feed contained no coccidiostatics nor antibiotics. Control upon arrival showed that they were EPEC, *Clostridium spiroforme,* rotavirus and *Eimeria* free (37, 48). Experimental rooms were desinfected prior to use, as were the individual cages. Rabbits were fed with commercial food free from anticoccidiostatics and food uptake and daily weigth gain were followed regularly. Experimental infection was done by giving *per os* 1 ml of a cell suspension grown overnight and diluted in phosphate buffered saline (PBS) to 1,6 x 10⁴ CFU's per ml. One of two rabbits was euthanized 7 days after infection, the second 10 days after infection. Tissue samples from duodenum, jejunum, ileum and caecum were taken for light microscope and electron microscope study (36).

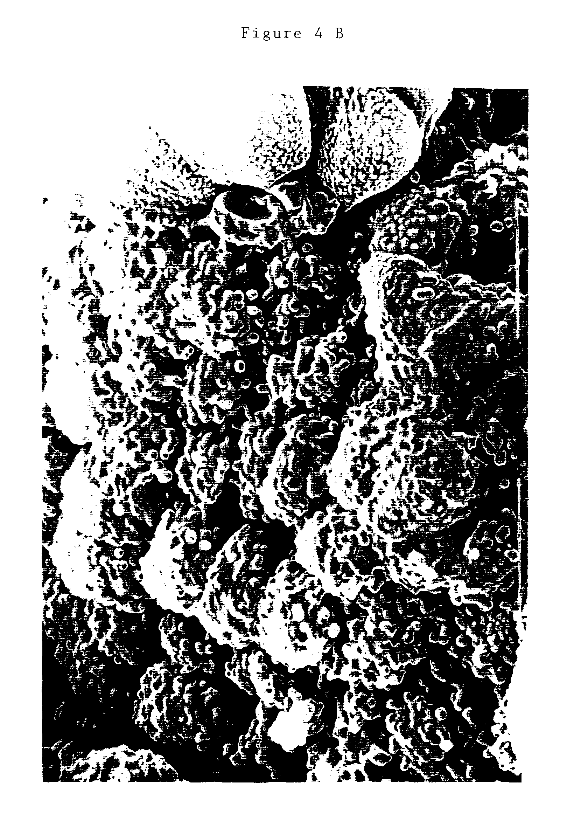

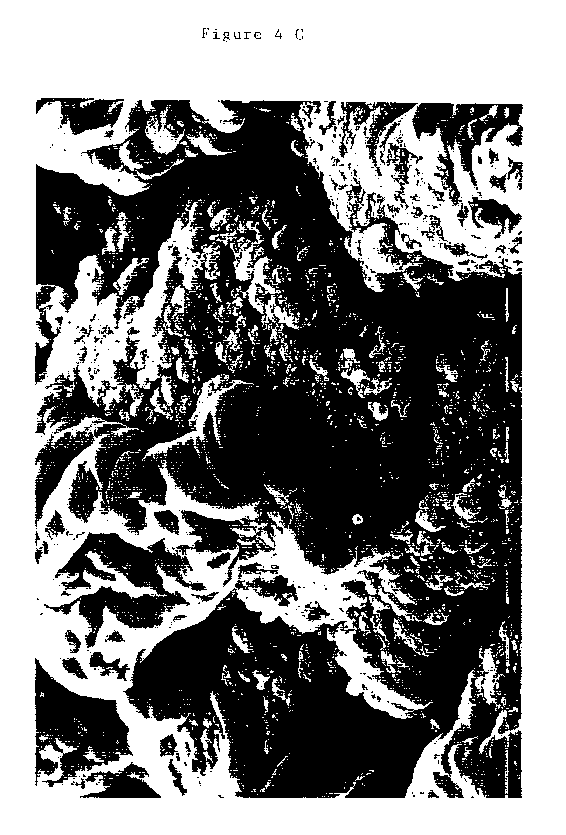

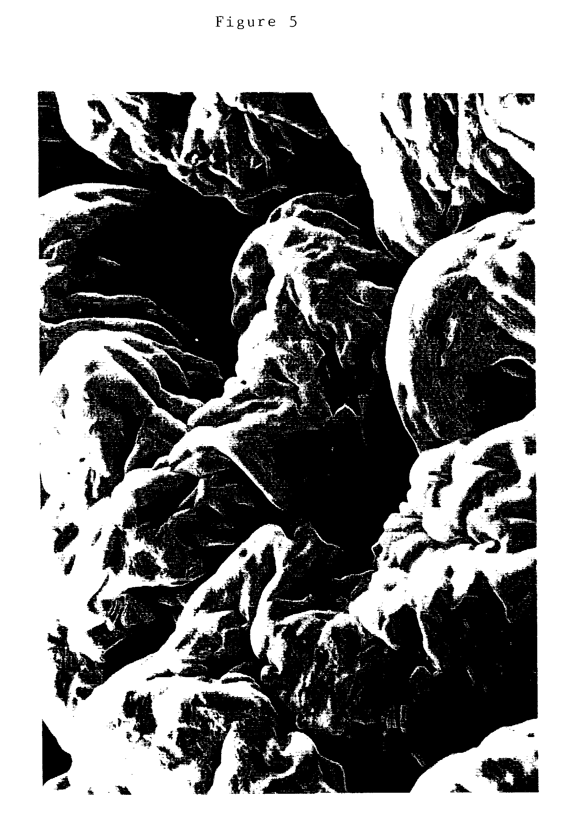

Clinical observations showed mild (97/223.10), light (97/241.6) and no diarrhea (control and REPEC O15:H- *Δeae*). Infection doses were rather low, because death caused by colibacillosis would interfere with clear histological observations using the microscope. Upon infection with 97/241.6 and 97/223.10, the feed conversion rate was clearly decreased, especially for the 97/223.10 infected rabbits. The feed conversion rate of REPEC O15:H- *Δeae* infected rabbits was not negatively influenced compared to the controle rabbits. Histological studies showed that 97/223.10 attached intimately, pedestals were seen and microvilli were effaced, resulting in clearly destructed cells in surrounding regions. These effects were stronger visible in the caecum, but also detectable in the ileum (see Figure 4). Infection with 97/241.6 lead to identical observations. Infection with REPEC O15:H- *Δeae* did however show that the strain was not attached intimately, but appeared concentrated in microcolonies in the lumen (see Figure 5). Tissue taken from control rabbits did not show attached organisms. Histological findings were observed by following the methods described in (36).

### Immunoprotection by using the mutant strain in a vaccine

A vaccination experiment was performed to observe the immunoprotective features of REPEC O15:H- *Δeae* against an infection with 97/223.10. 42 Rabbits were purchased, controlled, housed and followed as described above. They were divided in three groups with 14 rabbits each, in a way that the average weigth of the three groups were highly similar. The first group was experimentally vaccinated *per os* with 1 ml of a PBS solution with 2,5 x 10⁵ CFU's/ml REPEC O15:H- *Δeae.* One week later the same group, together with the second group was experimentally infected with 97/223.10 (5,4 x 10⁴ CFU's /ml). The third group served as control group and was never infected with an EPEC strain.

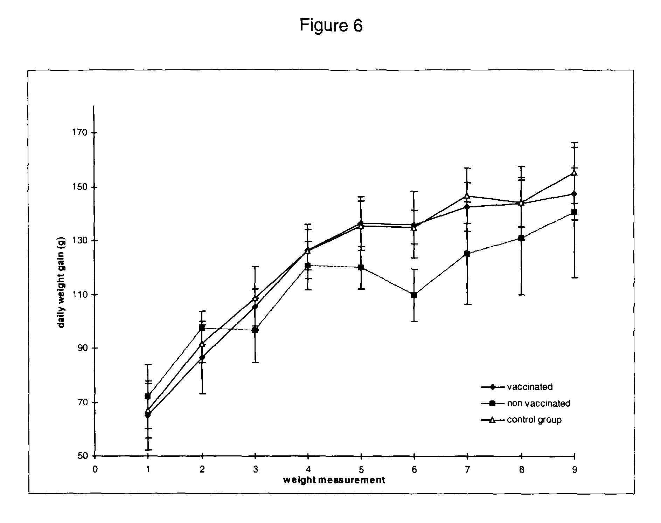

The group of non vaccinated animals suffered more from diarrhea then the vaccinated rabbits. Even though the infection dose was low, one of the non-vaccinated animals died from colibacillosis as it was the only possibility upon autopsy. Non vaccinated animals showed a delay in weigth gain compared to vaccinated and control animals. Also the daily feed uptake differed between vaccinated and non-vaccinated animals, as feed uptake of non-vaccinated animals is reduced to a significantly different level (alfa = 0,05) compared to control and vaccinated rabbits (see Figure 6). When feed conversions are calculated, no significant differences are found with the Student t-test between the three groups before infection with 97/223.10 (vaccinated to control: p=0,52; vaccinated to non-vaccinated: p=0,35; control to non-vaccinated: p=0,73). Following infection of group 1 and 2 , feed conversions differ clearly for non-vaccinated and vaccinated rabbits (vaccinated to control: p=0,68; vaccinated to non-vaccinated: p=0,02; control to non-vaccinated: p=0,02). The feed conversion ratio of the non-vaccinated rabbits is significantly lower compared to the ratio's of vaccinated and controle strains. No significant difference was found between vaccinated and control groups.

### The obtained strain as a basis for further vaccine investigation

Further study of the strain learns us more about the signalling between the pathogenic organism and its host. Adhesion characteristics are studied *in vitro* on different cell lines. This, together with sequencing of the region surrounding the deleted 248 bp region gives more clarity about the precise features of REPEC O15:H-Δ*eae*. The strain is tested for its precise immunogenic and protective role by following humoral and T-cell responses. Additional modifications are tested for their immunogenic effect.

### REFERENCES

1. **Abe, A., U. Heczko, R. G. Hegele, and B. Brett Finlay.** 1998. Two Enteropathogenic Escherichia coli Type III Secreted Proteins, EspA and EspB, Are Virulence Factors. J Exp Med. **188**:1907-1916.
2. **Abe, A., B. Kenny, M. Stein, and B. B. Finlay.** 1997. Characterization of two virulence proteins secreted by rabbit enteropathogenic Escherichia coli, EspA and EspB, whose maximal expression is sensitive to host body temperature. Infect Immun. **65**:3547-55.
3. **Adams, L. M., C. P. Simmons, L. Rezmann, R. A. Strugnell, and R. M. Robins-Browne.** 1997. Identification and characterization of a K88- and CS31A-like operon of a rabbit enteropathogenic Escherichia coli strain which encodes fimbriae involved in the colonization of rabbit intestine. Infect Immun. **65**:5222-30.
4. **Agin, T. S., J. R. Cantey, E. C. Boedeker, and M. K. Wolf.** 1996. Characterization of the eaeA gene from rabbit enteropathogenic Escherichia coli strain RDEC-1 and comparison to other eaeA genes from bacteria that cause attaching-effacing lesions. FEMS Microbiol Lett. **144**:249-58.
4a. **Boury, M., K. Grange, J.P. Nougayrede, E. Oswald, J. De Rycke and A. Milon.** 1998. Mutants du Locus of Enterocyte Effacement de Escheridia coli entéropathogènes du lapin et vaccination orale contre la collibacillose O103., P. 65-68. 7ièmes Journées de la Récherche Cunicole en France. ITAVI, 28 rue du Rocher, 75008 Paris, Lyon, France.
5. **Camguilhem, R., and A. Milon.** 1990. Protection of weaned rabbits against experimental Escherichia coli 0103 intestinal infection by oral formalin-killed vaccine. Vet Microbiol. **21**:353-62.
6. **Cantey, J. R., and R. K. Blake.** 1977. Diarrhea due to Escherichia coli in the rabbit: a novel mechanism. J Infect Dis. **135**:454-62.
7. **Cantey, J. R., L. R. Inman, and R. K. Blake.** 1989. Production of diarrhea in the rabbit by a mutant of Escherichia coli (RDEC-1) that does not express adherence (AF/R1) pili. J Infect Dis. **160**:136-41.
8. **Donnenberg, M. S., and J. B. Kaper.** 1991. Construction of an eae deletion mutant of enteropathogenic Escherichia coli by using a positive-selection suicide vector. Infect Immun. **59**:4310-7.
9. **Donnenberg, M. S., and J. B. Kaper.** 1992. Enteropathogenic Escherichia coli. Infect Immun. **60**:3953-61.
10. **Donnenberg, M. S., C. O. Tacket, S.P. James, G. Losonsky, J. P. Nataro, S.S. Wasserman, J. B. Kaper, and M. M. Levine.** 1993. Role of the *eae*A gene in experimental enteropathogenic Escherichia coli infection. J Clin Invest **92**:1412-7.
11. **Elliott, S. J., L. A. Wainwright, T. K. McDaniel, K. G. Jarvis, Y. K. Deng, L. C. Lai, B. P. McNamara, M. S. Donnenberg, and J. B. Kaper.** 1998. The complete sequence of the locus of enterocyte effacement (LEE) from enteropathogenic Escherichia coli E2348/69. Mol Microbiol. **28**:1-4.
12. **Fiederling, F., M. Boury, C. Petit, and A. Milon.** 1997. Adhesive factor/rabbit 2, a new fimbrial adhesin and a virulence factor from Escherichia coli 0103, a serogroup enteropathogenic for rabbits. Infect Immun. **65**:847-51.
13. **Giron, J. A., A. S. Ho, and G. K. Schoolnik.** 1991. An inducible bundle-forming pilus of enteropathogenic Escherichia coli. Science. **254**:710-3.
14. **Hicks, S., G. Frankel, J. B. Kaper, G. Dougan, and A. D. Phillips.** 1998. Role of intimin and bundle-forming pili in enteropathogenic Escherichia coli adhesion to pediatric intestinal tissue in vitro. Infect Immun. **66**:1570-8.
15. **Jarvis, K. G., J. A. Giron, A. E. Jerse, T. K. McDaniel, M. S. Donnenberg, and J. B. Kaper.** 1995. Enteropathogenic Escherichia coli contains a putative type III secretion system necessary for the export of proteins involved in attaching and effacing lesion formation. Proc Natl Acad Sci U S A. **92**:7996-8000.
16. **Jerse, A. E., J. Yu, B. D. Tall, and J. B. Kaper.** 1990. A genetic locus of enteropathogenic Escherichia coli necessary for the production of attaching and effacing lesions on tissue culture cells. PNAS. **87**:7839-7843.
17. **Karaolis, D. K., T. K. McDaniel, J. B. Kaper, and E. C. Boedeker.** 1997. Cloning of the RDEC-1 locus of enterocyte effacement (LEE) and functional analysis of the phenotype on HEp-2 cells. Adv Exp Med Biol. **412**:241-5.
18**. Kenny, B., R. DeVinney, M. Stein, D. J. Reinscheid, E. A. Frey, and B. B. Finlay.** 1997. Enteropathogenic E. coli (EPEC) transfers its receptor for intimate adherence into mammalian cells. Cell. **91**:511-20.
19. **Knutton, S., M. M. Baldini, J. B. Kaper, and A. S. McNeish.** 1987. Role of plasmid-encoded adherence factors in adhesion of enteropathogenic Escherichia coli to HEp-2 cells. Infect Immun. **55**:78-85.
20. **Knutton, S., I. Rosenshine, M. J. Pallen, I. Nisan, B. C. Neves, C. Bain, C. Wolff, G. Dougan, and G. Frankel.** 1998. A novel EspA-associated surface organelle of enteropathogenic Escherichia coli involved in protein translocation into epithelial cells. Embo J. **17**:2166-76.
21. **Link, A. J., D. Phillips, and G. M. Church.** 1997. Methods for generating precise deletions and insertions in the genome of wild-type Escherichia coli: application to open reading frame characterization. J Bacteriol. **179**:6228-37.
22. **McDaniel, T. K., K. G. Jarvis, M. S. Donnenberg, and J. B. Kaper.** 1995. A genetic locus of enterocyte effacement conserved among diverse enterobacterial pathogens. Proc Natl Acad Sci U S A. **92**:1664-8.
23. **Milon, A., and R. Camguilhem.** 1989. Essais de protection de lapereaux sevrés contre l'entérite à Escherichia coli O103: vaccinations des mères avec un vaccin inactivé. Revue de Médecine Vétérinaire. **140**:389-395.
24. **MIlon, A., and J. De Rycke.** 1997. Souches mutantes non pathogenes d'E. coli, leur procédé d'obtention et leurs utilisations., Institut National de la Propriété Industrielle, Paris., France.
25. **Milon, A., J. Esslinger, and R. Camguilhem.** 1992. Oral vaccination of weaned rabbits against enteropathogenic Escherichia coli-like E. coli O103 infection: use of heterologous strains harboring lipopolysaccharide or adhesin of pathogenic strains. Infect Immun. **60**:2702-9.
26. **Moon, H. W., S. C. Whipp, R. A. Argenzio, M. M. Levine, and R. A. Giannella.** 1983. Attaching and effacing activities of rabbit and human enteropathogenic Escherichia coli in pig and rabbit intestines. Infect Immun. **41**:1340-51.
27. **Nataro, J. P., and J. B. Kaper.** 1998. Diarrheagenic Escherichia coli [published erratum appears in Clin Microbiol Rev 1998 Apr;11(2):403]. Clin Microbiol Rev. **11**:142-201.
28. **Nisan, I., C. Wolff, E. Hanski, and I. Rosenshine.** 1998. Interaction of enteropathogenic Escherichia coli with host epithelial cells. Folia Microbiol. **43**:247-52.
29. **Nougayrede, J. P., O. Marches, M. Boury, J. Mainil, G. Charlier, P. Pohl, J. De Rycke, A. Milon, and E. Oswald.** 1999. The long-term cytoskeletal rearrangement induced by rabbit enteropathogenic Escherichia coli is Esp dependent but intimin independent [In Process Citation]. Mol Microbiol. **31**:19-30.
30. **Okerman, L., and L. A. Devriese.** 1985. Biotypes of enteropathogenic Escherichia coli strains from rabbits. J Clin Microbiol. **22**:955-8.
31. **Orskov, F., and I. Orskov.** 1984. Serotyping of Escherichia coli ., p. 43-112. *In* T. Bergan (ed.), Methods in Microbiology, vol. 14. Academic Press, Inc., London.
32. **Paton, A. W., P. A. Manning, M. C. Woodrow, and J. C. Paton.** 1998. Translocated intimin receptors (Tir) of shiga-toxigenic escherichia coli isolates belonging to serogroups O26, O111, and O157 react with sera from patients with hemolytic-uremic syndrome and exhibit marked sequence heterogeneity. Infect Immun. **66**:5580-6.
33. **Peeters, J., R. Geeroms, and B. Glorieux.** 1984. Experimental Escherichia coli enteropathy in weanling rabbits: clinical manifestations and pathological findings. Journal of Comparative Pathology. **94**:521-528.
33a. **Peeters, J. E., P. Phol, L. Okerman and L. A. Devriese.** 1984. Pathogenic properties of Escheridia coli strains isolated from diarrheie commercial rabbits. J Clin Microbiol **20**:34-9.
34. **Peeters, J. E.** 1994. E. coli infections in animals, p. 261-283. *In* G. C.L. (ed.), Escherichia coli in domestic animals and humans. CAB International, Wallingford, UK.
35. **Peeters, J. E., G. J. Charlier, and P. H. Halen.** 1984. Pathogenicity of attaching effacing enteropathogenic Escherichia coli isolated from diarrheic suckling and weanling rabbits for newborn rabbits. Infect Immun. **46**:690-6.
36. **Peeters, J. E., G. J. Charlier, and R. Raeymaekers.** 1985. Scanning and transmission electron microscopy of attaching effacing Escherichia coli in weanling rabbits. Vet Pathol. **22**:54-9.
37. **Peeters, J. E., R. Geeroms, R. J. Carman, and T. D. Wilkins.** 1986. Significance of Clostridium spiroforme in the enteritis-complex of commercial rabbits. Vet Microbiol. **12**:25-31.
38. **Peeters, J. E., R. Geeroms, and F. Orskov.** 1988. Biotype, serotype, and pathogenicity of attaching and effacing enteropathogenic Escherichia coli strains isolated from diarrheic commercial rabbits. Infect Immun. **56**:1442-8.
39. **Peeters, J. E., P. Pohl, and G. Charlier.** 1984. Infectious agents associated with diarrhoea in commercial rabbits: a field study. Ann Rech Vet. **15**:335-40.
40. **Peeters, J. E., I. Villacorta, E. Vanopdenbosch, D. Vandergheynst, M. Naciri, E. Ares-Mazas, and P. Yvore.** 1992. Cryptosporidium parvum in calves: kinetics and immunoblot analysis of specific serum and local antibody responses (immunoglobulin A [IgA], IgG, and IgM) after natural and experimental infections. Infect Immun. **60**:2309-16.
41. **Pohl, P. H., J. E. Peeters, E. R. Jacquemin, P. F. Lintermans, and J. G. Mainil.** 1993. Identification of eae sequences in enteropathogenic Escherichia coli strains from rabbits. Infect Immun. **61**:2203-6.
42. **Rosenshine, I.** 1998. Species specificity and tissue tropism of EPEC and related pathogens. Trends Microbiol. **6**:388.
43. **Rosenshine, I., S. Ruschkowski, M. Stein, D. J. Reinscheid, S. D. Mills, and B. B. Finlay.** 1996. A pathogenic bacterium triggers epithelial signals to form a functional bacterial receptor that mediates actin pseudopod formation. Embo J. **15**:2613-24.
44. **Sambrook, J., E. F. Fritsch, and T. Maniatis.** 1989. Molecular cloning : a laboratory manual, 2nd ed. Cold Spring Harbor Laboratory, Cold Spring Harbor, N.Y.
45. **Taylor, K. A., C. B. O'Connell, P. W. Luther, and M. S. Donnenberg.** 1998. The EspB protein of enteropathogenic escherichia coli is targeted to the cytoplasm of infected HeLa cells [In Process Citation]. Infect Immun. **66**:5501-7.
46. **van Miltenburg, R., B. Ruger, S. Grunewald-Janho, M. Leons, and C. Schroder (ed.).** 1995. The DIG System User's Guide for Filter Hybridization. Boehirnger Mannheim GmbH, Mannheim.
47. **Vandekerchove, D., and J. Peeters.** 1998. Detection of specific antibodies against strains of enteropathogenic Escherichia coli (EPEC) in the rabbit by an ELISA using 94--kilodalton proteins. World Rabbit Science. **6**:303-310.
48. **Vanopdenbosch, E.** 1980. Immunodiffusion test for the detection of rotavirus antigen in faecal material and gut homogenates. Current Topics in Veterinary Medicine and animal Science. **13**:118-120.
49. **Wolff, C., I. Nisan, E. Hanski, G. Frankel, and I. Rosenshine.** 1998. Protein translocation into host epithelial cells by infecting enteropathogenic Escherichia coli. Mol Microbiol. **28**:143-55.

## Claims

1. An attenuated mammalian enteropathogenic *E*. *coli* (EPEC) strain comprising a deletion of an intimin encoding gene sequence wherein said deletion fully eliminates a functional intimin protein.

2. A strain according to claim 1 wherein said strain is a rabbit enteropathogenic *E. coli* (EPEC) strain comprising a deletion of an intimin encoding gene sequence wherein said deletion fully eliminates a functional intimin protein.

3. A strain according to any of claims 1 or 2 as deposited under No LMG P-18935 on April 22, 1999 in the Belgian Coordinated Collections of Microorganisms LMG collection.

4. A recombinant vector comprising a deletion of an intimin encoding gene sequence, wherein said deletion fully eliminates a functional intimin protein.

5. A vector according to claim 4 wherein said deletion consists of a deletion of the *eae* gene from a rabbit EPEC strain.

6. A method for producing a strain according to any of claims 1 to 3 comprising the step of introducing an *eae* gene deletion in a wild-type EPEC strain by homologous recombination using a vector according to any of claims 4 to 5.

7. An immunogenic composition comprising an attenuated EPEC strain of any of claims 1 to 3.

8. A vaccine composition which provides protective immunity against an EPEC infection in a mammal comprising an attenuated strain according to any of claims 1 or 3.

9. A vaccine composition according to claim 8 which is an oral vaccine.

10. A method for in vitro differentiating between field infected and vaccinated individuals wherein said vaccinated individuals have been vaccinated with a vaccine composition according to claim 8 or 9 and said field infected infected individuals are in vitro diagnosed on the basis of the presence of intimin antibodies.

11. A method according to claim 10 wherein differentiation between wild type and mutant strain is made by using SEQ ID numbers 3 and 4 in an amplification reaction.

12. Use of an attenuated EPEC strain according to any of claims 1 to 3 for the preparation of a vaccine.
